# EUROPEAN PATENT APPLICATION

(11) **EP 1 880 997 A1**
(43) Date of publication of application: **23.01.2008**
(21) Application number: 06746343.0
(22) Date of filing: 12.05.2006
(51) Int. Cl.: C07D 477/20, A61K 31/407, A61P 31/04

(54) **CRYSTAL OF 1-METHYLCARBAPENEM COMPOUND**

(30) Priority: 13.05.2005 JP 2005140683
(71) Applicant: Daiichi Sankyo Company, Limited, Chuo-ku Tokyo 103-8426 (JP)
(72) Inventor: MICHIDA, Makoto c/o Daiichi Sankyo Company, Ltd., Kanagawa 254-0014 (JP); KOBAYASHI, Satoshi c/o Daiichi Sankyo Company, Ltd., Kanagawa 254-0014 (JP)
(74) Representative: Gibson, Christian John Robert
(86) International application number: PCT/JP2006/309546
(87) International publication number: WO 2006/121151

(57) **Abstract**

Among crystalline forms of compound (I), a 1-methylcarbapenem compound represented by the formula (I) in a crystalline form that shows main peaks in the X-ray powder diffraction pattern obtained with copper K_{α} irradiation.

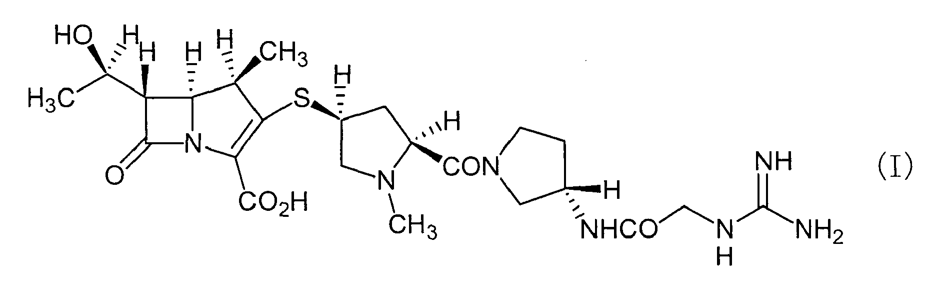

## Description

### [TECHNICAL FIELD]

The present invention relates to crystalline forms of a 1-methylcarbapenem compound that exhibit excellent antibacterial activity and storage stability, ease of handling and particularly superior solubility in water, pharmaceuticals containing these crystalline forms as active ingredient (and particularly pharmaceutical compositions for the prevention or treatment of bacterial infections), the use of these crystalline forms for the preparation of pharmaceuticals for the prevention or treatment of bacterial infections, the use of these crystalline forms for the prevention or treatment of bacterial infections, and methods for preventing or treating bacterial infections by administering pharmacologically effective amounts of these crystalline forms to a warm-blooded animal.

### [BACKGROUND ART]

Patent Document 1 and Patent Document 2 disclose a 1-methylcarbapenem compound represented by the formula shown below. 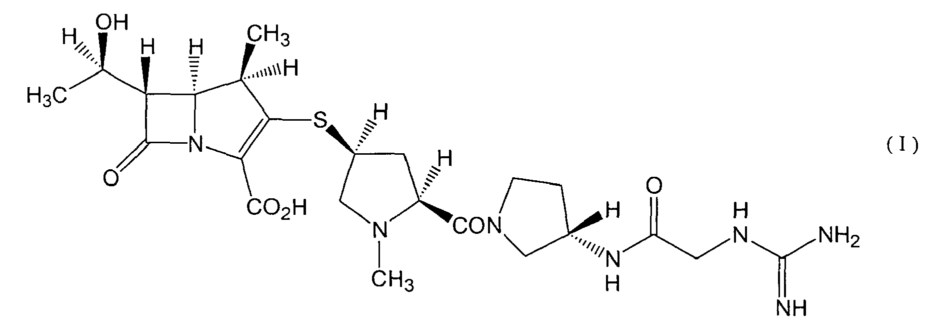 This compound (I) demonstrates excellent antibacterial activity not only against Gram-negative bacteria but also against Gram-positive bacteria, and is expected to be useful as an antibacterial agent.

Patent Document 3 and Patent Document 4 disclose specific crystalline forms of compound (I) and pharmacologically acceptable salts thereof. Although these crystalline forms have better storage stability and are handled more easily than freeze-dried powders, they still cannot be said to be completely without problems with respect to storage stability, handling ease and especially solubility in water.
[Patent Document 1] Japanese Patent Application (Kokai) No. Hei 10-204086
[Patent Document 2] Japanese Patent Application (Kokai) No. Hei 11-71277
[Patent Document 3] Japanese Patent Application (Kokai) No. 2001-72681
[Patent Document 4] Japanese Patent Application (Kokai) No. 2002-161034

### [DISCLOSURE OF THE INVENTION]

### [Problems to be Solved by the Invention]

It is important that bulk drugs of pharmaceuticals can be stored for long periods of time while maintaining their quality. Since large-scale refrigeration equipment is required to maintain quality in cases when storage at low temperatures is required, it is industrially significant to find stable crystalline forms that can be stored at room temperature. In addition, in the case of developing a pharmaceutical in the form of an injection preparation in particular, it is preferable that crystalline forms rapidly dissolve in water both during the formulation process at the factory and during administration of a preparation to a patient. On the basis of these factors, it is believed that the discovery of crystalline forms having storage stability while rapidly dissolving in water would make it possible to further improve the productivity and convenience of pharmaceuticals.

Therefore, as a result of conducting various studies to solve these problems, the inventors of the present invention succeeded in obtaining specific, novel crystalline forms of a 1-methylcarbapenem compound. These crystalline forms were found to have storage stability equal to or better than that of conventionally known crystalline forms, to have superior solubility in water, and to be extremely excellent as a bulk drug (especially a bulk drug for injection preparations) of medicaments (especially antibacterial agents), thereby leading to completion of the present invention.

### [Means for Solving the Problems]

The present invention relates to:
(1) a 1-methylcarbapenem compound represented by the aforementioned formula (I) in the crystalline form that shows main peaks at interplanar spacings d = 13.30, 9.86, 8.17, 7.00 and 4.59 in the X-ray powder diffraction pattern obtained with copper K_{α} irradiation;
(2) a 1-methylcarbepenem compound acetonate represented by the formula (I-1) in the crystalline form that shows main peaks at interplanar spacings d = 13.30, 9.86, 8.17, 7.00 and 4.59 in the X-ray powder diffraction pattern obtained with copper K_{α} irradiation; and, 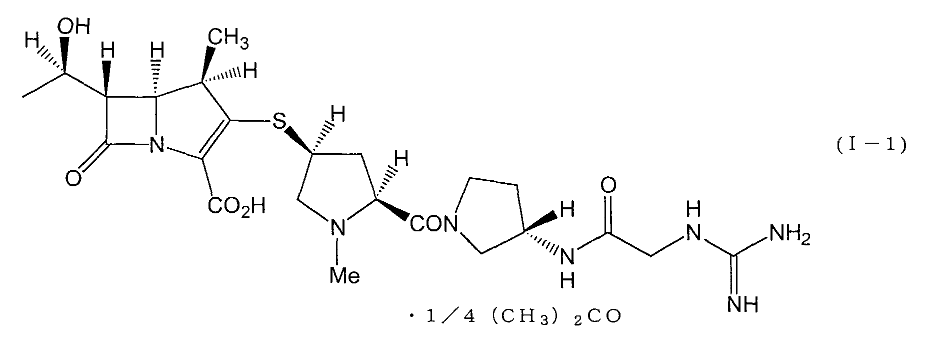
(3) pharmaceutical compositions, and particularly antibacterial agents, comprising the aforementioned crystalline forms as an active ingredient thereof.

In the above descriptions,
the 1-methylcarbapenem compound represented by formula (I) is disclosed in Japanese Patent Application Publications (Kokai) Nos. Hei 10-204086 and Hei 11-071277, and exhibits potent antibacterial activity against a broad spectrum of bacterial strains ranging from Gram-positive to Gram-negative bacterial strains.

The compound represented by formula (I-1) is an acetonate of compound (I).

The crystalline forms of the present invention can be produced by dissolving a 1-methylcarbapenem compound represented by formula (I), or a salt thereof, in for example, a water-acetone solution followed by the addition of a base as necessary and precipitation of crystals from this solution.

Salts of compound (I) can be acid addition salts including inorganic acid salts such as hydrochloride, hydrobromide, sulfate and phosphate; organic acid salts such as carbonate, acetate, benzoate, oxalate, maleate, fumarate, tartrate and citrate; sulfonates such as methanesulfonate, benzenesulfonate and p-toluenesulfonate; and base addition salts including alkaline metal salts such as sodium salt, potassium salt and lithium salt; metal salts such as calcium salt, magnesium salt and cobalt salt; and quaternary ammonium salts such as ammonium salt.

In the present invention, compound (I) may absorb moisture or adsorb water when allowed to stand in air, to form a hydrate, or may absorb certain other types of solvents resulting in the formation of a solvate. Such hydrates and solvates are also included in compound (I).

In the present invention, a hydrate or solvate of compound (I) is preferably a hydrate or acetonate, and more preferably an acetonate.

The crystalline forms of the present invention refer to solids in which the internal structure thereof consists of regularly repeating three-dimensional constituent atoms (or groups thereof), and are distinguished from amorphous solids not having such regular internal structure.

Crystalline forms having a plurality of different internal structures or physicochemical properties may be formed even for crystalline forms of the same compound depending on crystallization conditions, and the crystalline forms of the present invention may include any of these crystalline forms or a mixture of two or more types thereof.

The crystalline forms of the present invention include crystalline forms of compound (I) containing only a small amount of the crystalline forms of the present invention, and preferably the crystalline forms of the present invention are crystalline forms of compound (I) that contain 50% or more, more preferably 70% or more, and further more preferably 80% or more, of the crystalline forms of the present invention.

The crystalline form of the 1-methylcarbapenem compound represented by formula (I-1) shows main peaks at interplanar spacings d = 13.30, 9.86, 8.17, 7.00 and 4.59 in the X-ray powder diffraction pattern obtained with copper K_{α} irradiation (wavelength λ = 1.54 Angstroms).

### [Effects of the Invention]

The crystalline forms of the present invention have improved storage stability and solubility in water among crystalline forms of compound (I), and are useful for industrial production.

### [BEST MODE FOR CARRYING OUT THE INVENTION]

The 1-methylcarbapenem compound represented by formula (I) can be prepared by the same technique as described in Japanese Patent Application Publications (Kokai) Nos. Hei 10-204086, Hei 11-071277 and 2002-212183, or by a similar procedure.

The crystalline forms of the present invention can be obtained, for example, by dissolving compound (I) or a salt thereof in an appropriate solvent (good solvent), concentrating as necessary, adding a poor solvent as necessary and cooling the solution as necessary to provide a supersaturated solution, followed by precipitating crystals, isolating the precipitated crystals and drying the crystals.

Although precipitation of the crystals can be spontaneous in a reaction vessel, precipitation can also be initiated or accelerated by addition of crystalline seeds or by mechanical stimulus such as ultrasonic wave irradiation and scratching on the surface of the reaction vessel.

In the case of handling a solution of compound (I), the solution is usually treated at -10°C to 60°C to avoid decomposition of compound (I); preferably at 0°C to 25°C.

A preferred cooling temperature for crystallization of the compound is 0°C to 10°C.

Examples of methods for the concentration of solutions of compound (I) include concentrating by evaporating the solvent while heating at normal or reduced pressure using a rotary evaporator and the like, and concentrating using a reverse osmotic membrane. Examples of reverse osmotic membranes used in the concentration of aqueous solutions include polyacrylonitrile membranes, polyvinyl alcohol membranes, polyamide membranes and cellulose acetate membranes.

Examples of good solvents for compound (I) include water, dimethyl sulfoxide, dimethylformamide and methanol; preferably water.

Examples of poor solvents for compound (I) include acetone alone and mixed solvents of acetone and solvents such as alcohols having 2 to 4 carbon atoms such as ethanol, propanol or butanol; ketones such as methyl ethyl ketone; ethers such as diethyl ether or tetrahydrofuran; or esters such as methyl acetate or ethyl acetate; preferably acetone.

For the starting compound (I) or a salt thereof, a lyophilized powder or isolated form of existing crystalline forms may be used. Since the starting material can be purified by crystallization, a crude preparation containing compound (I) or a salt thereof can also be used.

Supersaturation can be accomplished, for example, by concentrating an aqueous solution of compound (I) at 30°C to 60°C to a saturated aqueous solution, followed by gradually cooling to 0°C to 10°C, or by gradual addition of a poor solvent such as acetone to the saturated aqueous solution, followed by cooling as necessary.

Preferably, the crystalline forms of the present invention are precipitated by concentrating an aqueous solution containing compound (I) as necessary, by addition of a poor solvent, and by cooling as necessary.

More preferably, the crystalline forms of the present invention are precipitated by concentrating an aqueous solution containing compound (I) as necessary, by addition of acetone and by cooling as necessary.

The precipitated crystalline forms can be isolated, for example, by filtration, centrifugation or decantation. The isolated crystalline forms can be washed with a suitable solvent as necessary. Preferably the crystalline forms are washed with the solvent used for crystallization.

The isolated crystalline forms are dried usually at 10°C to 50°C, preferably at 20°C to 30°C, until the weight of the crystalline form becomes almost constant. The crystalline form can be dried in the presence of a drying agent such as silica gel or calcium chloride or under reduced pressure as necessary.

The crystalline forms obtained have improved storage stability at normal humidity and room temperature as compared with conventionally known hydrate crystalline forms, ethanolate crystalline forms or crystalline forms of compounds retaining both water and ethanol as solvates, and further have extremely superior solubility in water as compared with other conventionally known crystalline forms.

The compound represented by formula (1-1) is a one-fourth acetonate, and although the weight of crystalline forms thereof becomes constant by drying at 45°C and 2 Pa for 60 hours, crystalline forms in any state from the non-dried state immediately after filtration to completion of drying are included in the present invention provided they exhibit the same X-ray powder diffraction pattern.

The crystalline forms of the present invention exhibit a wide antibacterial spectrum and potent antibacterial activity against Gram-positive strains, Gram-negative strains and anaerobic bacteria, as well as bacteria producing cephalosporinase. When antibacterial activity is determined by the agar-plate dilution method, they exhibit potent antibacterial activity against a broad range of pathogenic bacteria, including Gram-positive strains such as *Staphylococcus aureus*, methicillin-resistant *Staphylococcus aureus, Streptococcus pneumoniae* and Enterococcus species; Gram-negative strains such as *Escherichia coli, Bacillus dysenteriae, Klebsiella pneumoniae,* Proteus vulgaris, Serratia, Enterobacteriaceae and *Pseudomonas aeruginosa;* and anaerobic bacteria such as *Bacteroides fragilis.* Moreover, the crystalline forms exhibit a potent antibacterial activity against *Helicobacter pylori,* which is often detected in patients with chronic gastritis and peptic ulcers.

When administered to mice after dissolving in an appropriate solvent, the crystalline forms of the present invention exhibit longer half-value periods of blood concentration as compared with conventional drugs of the same type, and exhibit good urinary recovery.

In addition, when subcutaneously administered to mice systemically infected with *Staphylococcus aureus, Streptococcus pneumoniae, Escherichia coli* or *Pseudomonas aeruginosa,* the crystalline forms of the present invention exhibit excellent treatment effects against the infections. Thus, the crystalline forms of the present invention are useful as medicaments (especially antibacterial agents) and also as bulk powders for the production thereof.

When the crystalline forms of the present invention are used as medicaments (especially antibacterial agents), they can be administered alone or mixed with suitable, pharmacologically acceptable excipients or diluents and the like and administered orally in the form of, for example, tablets, capsules, granules, powders or syrup, parenterally by injection and the like, or topically in the form of ointments and the like.

Such preparations are prepared by methods known to those skilled in the art using additives such as excipients (for example, sugar derivatives such as lactose, sucrose, glucose, mannitol and sorbitol; starch derivatives such as corn starch, potato starch, α-starch, dextrin and carboxymethylstarch; cellulose derivatives such as crystalline cellulose, low-substituted hydroxypropylcellulose, hydroxypropylmethyl cellulose, carboxymethylcellulose, calcium carboxymethyl cellulose and internally-crosslinked sodium carboxymethylcellulose; arabic gum; dextran; pullulan; silicate derivatives such as light anhydrous silicic acid, synthetic aluminium silicate and magnesium metasilicate aluminate; phosphate derivatives such as calcium phosphate; carbonate derivatives such as calcium carbonate; and sulfate derivatives such as calcium sulfate), binders (for example, excipients as described above; gelatin; polyvinylpyrrolidone; and macrogol), disintegrants (for example, excipients as described above, and chemically modified starch and cellulose derivatives such as cross-carmellose sodium, sodium carboxymethylstarch and crosslinked polyvinylpyrrolidone), lubricants (for example, talc; stearic acid; metal stearates such as calcium stearate and magnesium stearate; colloidal silica; bee gum; waxes such as beeswax and spermaceti; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as anhydrous silicic acid and silicic acid hydrate; and starch derivatives as described for the excipients), stabilizers (for example, paraoxybenzoic acid esters such as methylparaben and propylparaben; alcohols such as chlorobutanol, benzyl alcohol and phenethyl alcohol; benzalkonium chloride; phenols such as phenol and cresol; thimerosal; acetic anhydride; and sorbic acid), corrigents (for example, sweetening, souring and flavouring agents all of which are usually used), suspending agents (for example, Polysorbate 80 and sodium carboxymethyl cellulose), diluents, solvents for formulation (for example, water, ethanol and glycerin), assisting agents for dissolution (for example, non-ionic surfactants and anionic surfactants), and topical anaesthetic agents (for example, lidocaine hydrochloride and mepivacaine hydrochloride).

Examples of dosage forms suitable for oral administration include solid preparations such as tablets, coated tablets, capsules, lozenges, powders, grains, granules and dry syrups, and liquid preparations such as syrups. Examples of dosage forms suitable for parenteral administration include injections, dripping infusions and suppositories. In addition, examples of dosage forms suitable for local administration include ointments, tinctures, creams and gels. These preparations can be prepared according to methods known in the field of pharmaceutical technology.

Preferred dosage forms of the crystalline 1-methylcarbapenem compounds of this invention are injections and dripping infusions. Suitable dosage levels for the crystalline forms depend on the age, body weight and symptoms of the patient and are usually from 10 mg (preferably 50 mg) to 6000 mg (preferably 4000 mg) for an adult human per day, which dosage can be administered as a single dose or divided into 2 to 4 doses through the day.

### [Examples]

Although the following provides a more detailed explanation of the present invention through examples, test examples and preparation examples thereof, the present invention is not limited thereto.

### Example 1

(1R,5S,6S)-6-[(1R)-1-Hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid acetonate

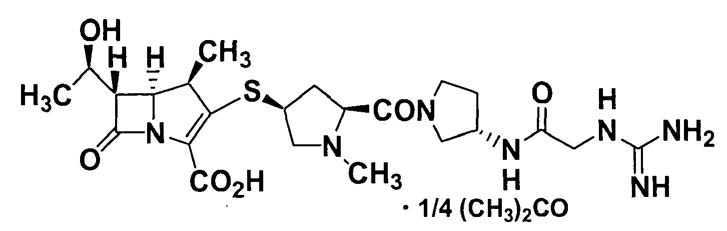

After suspending (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (50.0 g) in water (500 ml), 1N hydrochloric acid was added to this suspension to adjust to pH 5 and dissolve the crystals. After adjusting the pH of this solution to pH 9 with a 1N aqueous sodium hydroxide solution, 2.5 g of activated charcoal was added followed by stirring for 10 minutes at room temperature. After filtering off the activated charcoal, acetone (2250 ml) was dropped into the reaction liquid at room temperature followed by cooling to 0°C and stirring for 1 hour. The precipitated crystals were filtered off and washed with a 75% aqueous acetone solution and acetone followed by drying under reduced pressure to give the target compound (41.20 g). Yield: 82.4%

Infrared absorption spectrum (KBr) νmax(cm⁻¹): 3409, 3345, 3275, 3185, 2967, 2884, 1761, 1674, 1644, 1586, 1551, 1452, 1415, 1380, 1369, 1340, 1282, 1254.

Nuclear magnetic resonance spectrum (400 MHz, DMSO) δ ppm: 1.13-1.24 (4.5H, m), 1.30 (3H, d, J=6.4 Hz), 1.57-1.72 (1H, m), 1.93-2.10 (1H, m), 2.15-2.35 (1H, m), 2.27,2.29 (3H, sX2), 2.68-2.88 (2H, m), 3.09 (1H, d, J=10.6 Hz), 3.29-3.73 (7H, m), 3.75-3.93 (2H, m), 4.01 (2H, s), 4.12-4.30 (2H, m), 4.38-4.50 (1H, m).

X-ray powder diffraction (Cu Kα, λ=1.54 Angstroms) d (Angstroms): 13.30, 9.86, 8.17, 7.00, 4.59.

### Example 2

(1R,5S,6S)-6-[(1R)-1-Hydroxethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbepen-2-em-3-carboxylic acid acetonate

Water (258 ml) was added to a tetrahydrofuran solution (372 ml) of (1R,5S,6S)-6-[(1R)-1-hydroxethyl]-1-methyl-2-[(2S,4S)-2-[(3S)-3-[2-[3-(4-nitrobenzyloxycarbonyl)guanidino]acetylamino]pyrrolidin-1-ylcarbonyl]-1-methylpyrrolidin-4-ylthio]-1-carbepen-2-em-3-carboxylic acid 4-nitrobenzyl ester]-1-carbepen-2-em-3-carboxylic acid 4-nitrobenzyl ester (46.68 g) followed by the addition of ethyl acetate (140 ml), 7.5% palladium carbon (12.92 g) and activated charcoal (9.34 g). After stirring this reaction liquid for 4 hours at room temperature in the presence of flowing hydrogen (3 kPa), nitrogen substitution was performed and the reaction liquid was stirred for 40 minutes while cooling with ice. The reaction liquid was filtered followed by washing with a 66% aqueous tetrahydrofuran solution (282 ml). The resulting filtrate was washed with ethyl acetate (560 ml) followed by concentrating the aqueous layer to 308 ml under reduced pressure. After adding activated charcoal (3.73 g) to the resulting aqueous layer, the mixture was stirred for 40 minutes at 5°C. After filtering off the activated charcoal and washing with water (93 ml), water (30 ml) was poured into the filtrate. After adjusting the pH of the resulting aqueous solution to pH 8.9 with 1N aqueous sodium hydroxide solution, acetone (616 ml) was dropped in at room temperature. Seed crystals (70 mg) were added to this solution, and after stirring for 1 hour at room temperature, acetone (616 ml) was further dropped in followed by stirring for 1 hour at the same temperature and then stirring for 1 hour while cooling with ice. The precipitated crystals were filtered off, washed with a 75% aqueous acetone and then dried under reduced pressure to give the title compound (21.03 g). Yield: 78.9%

### Test Example 1

### Storage Stability Test - 1

The crystalline form of the present invention obtained according to Example 2 and a crystalline form described in Japanese Patent Application Publication (Kokai) No. 2001-72681 ((1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbepen-2-em-3-carboxylic acid monoethanolate - trihydrate, referred to as Crystalline form 1) were sealed in a brown glass bottle at a constant temperature of 25°C followed by measurement of purity at the start of the test, one month later and two months later using high performance liquid chromatography (column: Develosil RPAQUEOUS 4.6 x 150 mm, column temperature: 60°C, developer flow rate: 1.0 ml/min, developer: 0.02 M aqueous potassium dihydrogen phosphate solution/acetonitrile = 95/5 to 50/50). The resulting purities were represented as the ratios of the areas of the chromatographic peaks. Those results are shown in Table 1.

**Table 1**

| Storage Stability Test at Room Temperature | | | |
|---|---|---|---|
| | Purity (%) | | |
| Test Crystalline form | Start of test | 1 month later | 2 months later |
| Crystalline form of present invention | 98.35 | 98.43 | 98.40 |
| Crystalline form 1 | 98.45 | 98.35 | 98.06 |

### Test Example 2

### Storage Stability Test - 2

The crystalline form of the present invention obtained according to Example 2 and Crystalline form 1 were sealed in a brown glass bottle at a constant temperature of 40°C followed by measurement of purity at the start of the test and one month later using high performance liquid chromatography (column: Develosil RPAQUEOUS 4.6 x 150 mm, column temperature: 60°C, developer flow rate: 1.0 ml/min, developer: 0.02 M aqueous potassium dihydrogen phosphate solution/acetonitrile = 95/5 to 50/50). The resulting purities were represented as the ratios of the areas of the chromatographic peaks. Those results are shown in Table 2.

**Table 2**

| Storage Stability Test at 40°C | | |
|---|---|---|
| Test Crystalline form | Purity at start of test (%) | Purity 1 month later (%) |
| Crystalline form of present invention | 98.35 | 98.35 |
| Crystalline form 1 | 98.45 | 98.26 |

### Test Example 3

### Water Solubility Test

The crystalline form of the present invention obtained according to Example 2 and a crystalline form described in Japanese Patent Application Publication (Kokai) No. 2001-72681 ((1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbepen-2-em-3-carboxylic acid, referred to as Crystalline form 2) were sized by passing through a 150 µm mesh filter followed by measurement of the amount of time required for 300 mg of the crystals to completely dissolve in 10 ml of water at 25°C. Those results are shown in Table 3.

**Table 3**

| Water Solubility Test | |
|---|---|
| Test Crystalline form | Dissolving time (min) |
| Crystalline form of present invention | 2.5 |
| Crystalline form 2 | 8.0 |

The crystalline form of the present invention demonstrates solubility in water superior to that of the known crystalline form.

### Preparation Example 1

### Injection Preparation

250 mg of the crystalline form of Example 1 was aseptically filled and sealed in a vial. This preparation can incorporate a known pharmaceutical additive including local anesthetic such as lidocaine hydrochloride. This preparation can be used by dissolving in a solvent such as distilled water at the time of administration, for injection.

### [BRIEF DESCRIPTION OF THE DRAWING]

[Fig. 1]

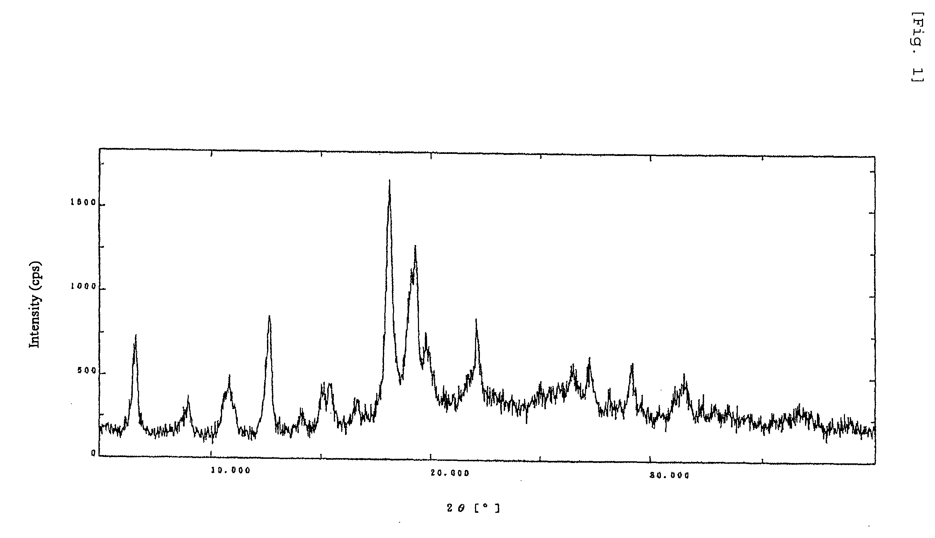

   Fig. 1 is an X-ray powder diffraction pattern obtained by irradiating crystalline form of (1R,5S,6S)-6-[(1R)-1-hydroxethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbepen-2-em-3-carboxylic acid acetonate (I-1) with copper K_{α} irradiation (wavelength λ = 1.54 Angstroms). Furthermore, the diffraction intensity in counts/sec (cps) is indicated on the vertical axis of the powder X-ray diffraction pattern, while the value of the diffraction angle 2θ is indicated on the horizontal axis.

### [INDUSTRIAL APPLICABILITY]

The crystalline forms of the present invention have improved storage stability and water solubility, and are extremely useful for practical use as medicaments and particularly as antibacterial agents.

## Claims

1. A 1-methylcarbapenem compound represented by the formula (I) in the crystalline form that shows main peaks at interplanar spacings d = 13.30, 9.86, 8.17, 7.00 and 4.59 in the X-ray powder diffraction pattern obtained with copper K_{α} irradiation.

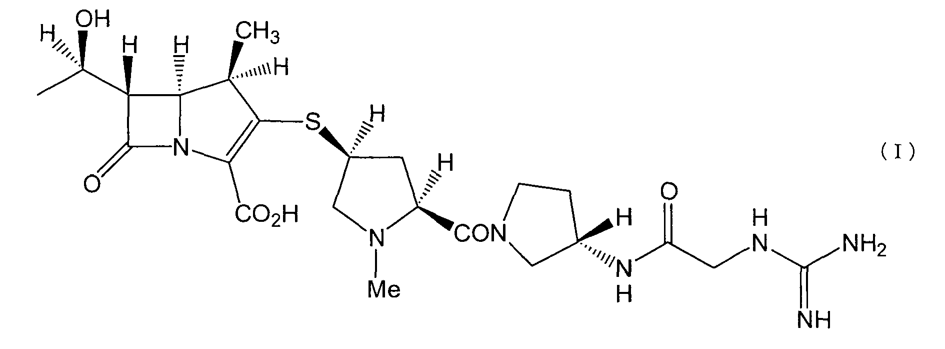

2. A 1-methylcarbepenem compound acetonate represented by the formula (I-1) in the crystalline form that shows main peaks at interplanar spacings d = 13.30, 9.86, 8.17, 7.00 and 4.59 in the X-ray powder diffraction pattern obtained with copper K_{α} irradiation.

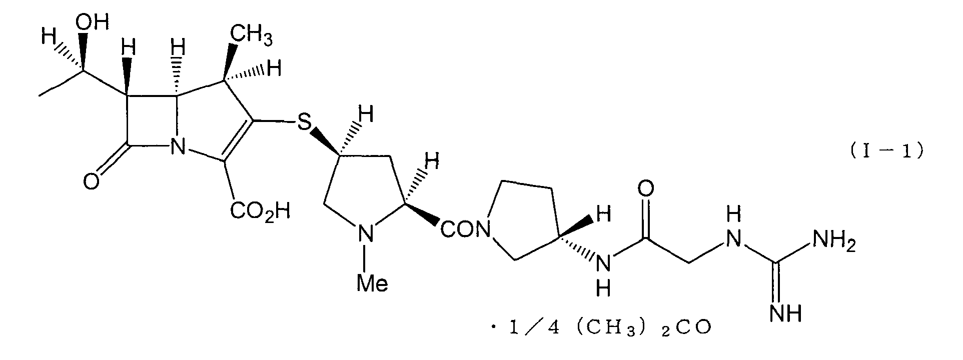

3. A pharmaceutical comprising the crystalline form according to claim 1 or 2 as an active ingredient thereof.

4. A pharmaceutical composition for the prevention or treatment of bacterial infections comprising the crystalline form according to claim 1 or 2 as an active ingredient thereof.

5. Use of the crystalline form according to claim 1 or 2 for the preparation of a pharmaceutical for the prevention or treatment of bacterial infections.

6. Use of the crystalline form according to claim 1 or 2 for the prevention or treatment of bacterial infections.

7. A method for preventing or treating bacterial infections by administering a pharmacologically effective amount of the crystalline form according to claim 1 or 2 to a warm-blooded animal.
